(19) Europäisches Patentamt
European Patent Office
Office européen des brevets

(11) Numéro de publication : **0 067 772 B1**

(12) # FASCICULE DE BREVET EUROPÉEN

(45) Date de publication du fascicule du brevet : **13.03.85**

(51) Int. Cl.[4] : **C 07 D417/12**, A 61 K 31/47

(21) Numéro de dépôt : **82401062.3**

(22) Date de dépôt : **11.06.82**

(54) **Nouveaux dérivés du N-dihydrothiazolyl 3-quinoléine carboxamide, leurs sels, leur procédé de préparation, leur application à titre de médicaments et les compositions les renfermant.**

(30) Priorité : **12.06.81 FR 8111607**
**15.03.82 FR 8204331**

(43) Date de publication de la demande :
**22.12.82 Bulletin 82/51**

(45) Mention de la délivrance du brevet :
**13.03.85 Bulletin 85/11**

(84) Etats contractants désignés :
**AT BE CH DE GB IT LI LU NL SE**

(56) Documents cités :
**EP-A- 0 012 639**
**FR-A- 1 364 605**
**FR-A- 2 281 761**
**FR-A- 2 324 304**
**FR-A- 2 340 735**
**FR-A- 2 377 400**
**JOURNAL OF HETEROCYCLIC CHEMISTRY, vol. 2, no. 2, juin 1965, pages 113-119, Pennsylvania (USA); A.S. DEY et al.: "Synthesis and properties of fluorine-containing heterocyclic compounds. I. Trifluoromethyl quinolines (1)"**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Clemence, François**
**2, rue Turgot**
**F-75009 Paris (FR)**
Inventeur : **Hunt, Peter Francis**
**6, avenue du Lycée**
**F-95500 Gonesse (FR)**
Inventeur : **Le Martret, Odile**
**42, avenue de Versailles**
**F-75016 Paris (FR)**
Inventeur : **Humbert, Daniel**
**15, rue Gaston Charle**
**F-94120 Fontenay-sous-Bois (FR)**

(74) Mandataire : **Douetteau, Pierre et al**
**c/o ROUSSEL-UCLAF 102, route de Noisy Boite postale 9**
**F-93230 Romainville (FR)**

**EP 0 067 772 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés du N-dihydrothiazolyl-3-quinoléine carboxamide ainsi que leurs sels, le procédé de préparation, l'application à titre de médicaments de ces nouveaux dérivés et les compositions les renfermant.

Le brevet français n° 2 340 735 décrit une large famille de dérivés N substitués du 4-hydroxy quinoléine-3-carboxamide dont le noyau quinoléine peut être substitué en position 5, 6, 7 ou 8 par divers substituants. Par ailleurs, le groupement carboxamide peut porter divers radicaux hétérocycliques dont par exemple, les radicaux thiazolyle et 4,5-dihydrothiazolyle.

Le seul dérivé spécifiquement décrit dans ce brevet, pour lequel l'azote du groupement amide est substitué par un radical 4,5-dihydrothiazolyle, comporte un radical trifluorométhyle en position 8 sur la quinoléine ; ce dérivé est doué d'activité analgésique, mais est dénué d'activité anxiolytique.

En effectuant des recherches sur cette série, la demanderesse vient de trouver qu'un groupe de dérivés du 4-hydroxy quinoléine 3-carboxamide dont la fonction amide est substituée par le radical 4,5-dihydrothiazolyle et dont le noyau quinoléine n'est pas substitué ou est substitué en position 6 ou 7, dérivés qui ne sont ni décrits, ni suggérés spécifiquement dans le brevet français précité, se trouvent dénués de propriétés analgésiques, mais sont par contre doués de propriétés anxiolytiques marquées. Ces dérivés se signalent par une forte affinité pour les récepteurs des benzodiazépines.

C'est ainsi que l'invention a pour objet de nouveaux dérivés du N-dihydrothiazolyl-3-quinoléine carboxamide ainsi que leurs sels d'addition avec les acides minéraux ou organiques, caractérisés en ce qu'ils répondent à la formule générale I :

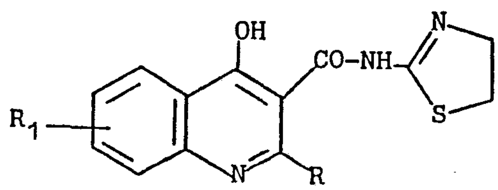

(I)

dans laquelle R représente un atome d'hydrogène ou un radical alcoyle renfermant de 1 à 4 atomes de carbones, et $R_1$ en position 6 ou 7, représente un atome d'hydrogène ou d'halogène un radical alcoyle linéaire renfermant de 1 à 4 atomes de carbone, un radical alcoyle ramifié renfermant de 3 à 5 atomes de carbone, un radical alcoxy renfermant de 1 à 4 atomes de carbone, un radical trifluorométhylthio, méthylthio, trifluoro méthoxy ou trifluorométhyle.

Dans la formule générale I et dans ce qui suit, lorsque R représente un radical alcoyle, il s'agit par exemple d'un radical propyle ou isopropyle, et de préférence d'un radical méthyle ou éthyle ; lorsque $R_1$ représente un radical alcoyle linéaire, il s'agit de préférence du radical méthyle, éthyle ou n-propyle ; lorsque $R_1$ représente un atome d'halogène, il s'agit de préférence d'un atome de fluor, de chlore, ou de brome ; lorsque $R_1$ représente un radical alcoyle ramifié, il s'agit de préférence du radical isopropyle ou isobutyle ; lorsque $R_1$ représente un radical alcoxy, il s'agit de préférence du radical méthoxy, éthoxy ou n-propoxy.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits objet de l'invention, on peut citer notamment les dérivés répondant à la formule I ci-dessus, ainsi que leurs sels d'addition avec les acides minéraux ou organiques, caractérisés en ce que, dans ladite formule I, R représente un atome d'hydrogène ou un radical méthyle, $R_1$ représente un atome d'hydrogène ou de chlore ou un radical méthoxy, ou $R_1$ en position 6, représente un atome de fluor, ou un radical méthyle, éthyle, isopropyle, méthylthio ou trifluorométhyle.

Parmi ces derniers, on peut citer les dérivés caractérisés en ce que dans ladite formule (I), R représente un atome d'hydrogène, et tout particulièrement le N-(4,5-dihydrothiazol-2-yl) 4-hydroxy 3-quinoléine carboxamide, le N-(4,5-dihydrothiazol-2-yl) 6-éthyl 4-hydroxy 3-quinoléine carboxamide, le N-(4,5-dihydrothiazol-2-yl) 6-chloro 4-hydroxy 3-quinoléine carboxamide, le N-(4,5-dihydrothiazol-2-yl) 4-hydroxy 2-méthyl 3-quinoléine carboxamide, ainsi que leurs sels d'addition avec les acides minéraux ou organiques.

L'invention a également pour objet un procédé de préparation des dérivés, tels que définis par la formule I ci-dessus ainsi que de leurs sels, caractérisé en ce que l'on fait réagir un produit de formule II

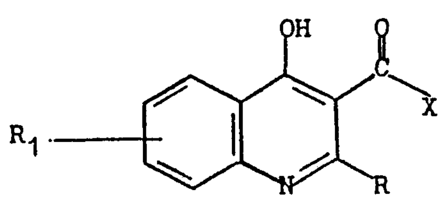

(II)

dans laquelle R et $R_1$ ont la signification déjà indiquée, et X représente un atome de chlore ou de brome, ou un radical hydroxy ou alcoxy, renfermant de 1 à 5 atomes de carbone, avec la 2-aminothiazoline, pour obtenir un produit de formule (I)

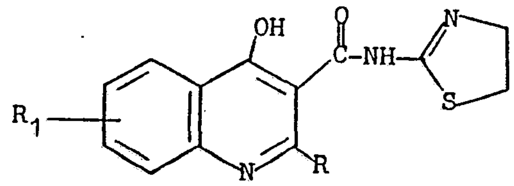

(I)

dans laquelle R et $R_1$ ont la signification déjà indiquée, que l'on isole et salifie si désiré.

La réaction d'un chlorure ou d'un bromure d'acyle de formule (II) avec la 2-aminothiazoline peut s'effectuer dans des solvants ou milieux de suspension inertes tels que les cétones aliphatiques inférieures, le dioxane, le diméthylformamide, le benzène, le toluène ou les halogénures d'alcoyle. On peut opérer avantageusement en présence d'agents fixateurs d'hydracides, tels que les hydroxydes alcalins comme la potasse, les carbonates alcalins,comme le carbonate de potassium, les bicarbonates alcalins comme ceux de sodium ou de potassium, les alcolates alcalins comme l'éthylate de sodium ou de préférence les amines tertiaires telles que les trialcoylamines ou la pyridine.

La réaction d'un acide de formule (II) avec la 2-aminothiazoline est avantageusement effectuée en présence de dicyclohexyl carbodiimide, au sein du diméthyl formamide.

On opère avantageusement à partir d'un ester d'alcoyle de formule (II), notamment un ester éthylique. On opère alors au reflux d'un solvant tel que ceux mentionnés ci-dessus à point d'ébullition élevé de 50 °C à 150 °C par exemple ; le reflux étant maintenu en général de 12 à 48 heures.

La réaction est effectuée de préférence en présence d'un agent de condensation tel qu'un dérivé alcoylé de l'aluminium, par exemple, le triméthylaluminium et notamment le triisobutylaluminium.

On peut également opérer avantageusement en présence de traces d'un hydrure alcalin tel que l'hydrure de sodium.

Les produits de formule (II) sont connus, ou peuvent être préparés comme indiqué dans le brevet français 2 340 735.

Les nouveaux dérivés de la N-dihydrothiazolyl-3-quinoléine carboxamide de formule I présentent un caractère basique. On peut avantageusement préparer leurs sels d'addition, en faisant réagir, en proportions sensiblement stœchiométriques, un acide minéral ou organique avec lesdits dérivés. Les sels peuvent être préparés sans isoler les bases correspondantes.

Les dérivés, objet de la présente invention, possèdent de très intéressantes propriétés pharmacologiques ; ils sont doués notamment d'une remarquable affinité pour les récepteurs des benzodiazépines. Ils possèdent une forte activité anxiolytique.

Ces propriétés sont illustrées plus loin dans la partie expérimentale.

Ces propriétés justifient l'utilisation des dérivés de la N-dihydrothiazolyl 3-quinoléine carboxamide de formule (I) ainsi que de leurs sels pharmaceutiquement acceptables, à titre de médicaments.

La présente demande a ainsi également pour objet l'application, à titre de médicaments, notamment anxiolytiques, des dérivés du N-dihydrothiazolyl 3-quinoléine carboxamide tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du N-dihydrothiazolyl-3-quinoléine carboxamide répondant à la formule (I), dans laquelle R représente un atome d'hydrogène ou un radical méthyle, $R_1$ représente un atome d'hydrogène ou de chlore ou un radical méthoxy, ou $R_1$, en position 6, représente un atome de fluor, ou un radical méthyle, éthyle, isopropyle, méthylthio, ou trifluorométhyle, ainsi que par leurs sels d'addition avec les acides pharmaceutiquement acceptables.

Parmi ceux-ci, on retient notamment ceux répondant à la formule (I), dans laquelle R représente un atome d'hydrogène, ainsi que leurs sels d'addition avec les acides pharmaceutiquement acceptables.

Parmi ces derniers, on retient tout particulièrement les dérivés dont les noms suivent :

— le N-(4,5-dihydrothiazol-2-yl) 4-hydroxy 3-quinoléine carboxamide,

— le N-(4,5-dihydrothiazol-2-yl) 6-éthyl 4-hydroxy 3-quinoléine carboxamide,

— le N-(4,5-dihydrothiazol-2-yl) 6-chloro 4-hydroxy 3-quinoléine carboxamide, ainsi que leurs sels d'addition avec les acides pharmaceutiquement acceptables. On retient également

— le N(4,5-dihydrothiazol-2-yl) 4-hydroxy 2-méthyl 3-quinoléine carboxamide, ainsi que ses sels d'addition avec les acides pharmaceutiquement acceptables.

Les médicaments selon l'invention trouvent leur emploi, par exemple dans le traitement des états anxieux tels que l'anxiété chronique, associée ou non à de l'insomnie ou à des troubles du comportement, de l'angoisse chez l'adulte ou l'enfant, ou en complément lors des traitements par neuroleptiques ou antidépresseurs dans les états psychotiques ou dépressifs.

La dose usuelle, variable selon le dérivé utilisé, le sujet de l'affection en cause peut être par exemple, de 1 mg à 100 mg par jour, par voie orale chez l'homme du dérivé de l'exemple 1 pour le traitement de l'anxiété chronique.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie orale ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent illustrent la présente invention, sans toutefois la limiter.

## Exemple 1

N-(4,5-dihydro-thiazol-2-yl) 4-hydroxy 3-quinoléine carboxamide et son chlorhydrate.

On agite 37,4 g de 2-aminothiazoline dans 800 $cm^3$ de chlorure de méthylène, ajoute en 20 mn, à 8 °C-10 °C, 167 $cm^3$ d'une solution toluénique de triisobutylaluminium à 25 %, agite encore 30 mn à 8 °C, ajoute 15,89 g de 3-carbéthoxy 4-hydroxy quinoléine, agite 30 mm, porte au reflux pendant 16 heures, évapore à sec sous pression réduite, reprend dans 1 litre d'une solution aqueuse normale d'acide chlorhydrique, filtre, glace pendant 16 heures, filtre le précipité et le lave à l'eau. On reprend les eaux mères, les alcalinise à l'aide de carbonate de sodium, filtre le précipité, le lave à l'eau, le sèche. On obtient 11,7 g de la base attendue.

Formation du chlorhydrate : on dissout le produit ci-dessus dans 600 $cm^3$ d'éthanol, filtre, ajoute 8 $cm^3$ d'une solution éthanolique d'acide chlorhydrique 5,3 N, glace, filtre, sèche et récupère 8,5 g de produit attendu. F ≃ 300 °C.

Analyse : $C_{13}H_{11}N_3O_2S$, HCl = 309,78

| | | | | | |
|---|---|---|---|---|---|
| Calculé : | C % 50,41 | H % 3,9 | N %13,56 | S % 10,35 | Cl % 11,44 |
| Trouvé : | C % 50,1 | H % 4,0 | N %13,3 | S % 10,4 | Cl % 11,2 |

## Exemple 2

Chlorhydrate de N-(4,5-dihydrothiazol-2-yl) 6-éthyl 4-hydroxy-3-quinoléine carboxamide.

On agite 10,2 g de 2-aminothiazoline dans 500 $cm^3$ de chlorure de méthylène, ajoute en 10 mn, à 7 °C-10 °C, 45,36 $cm^3$ d'une solution toluénique de triisobutylaluminium 1,1 M, agite encore 1 heure à 7 °C-10 °C, ajoute 4,88 g de 6-éthyl 4-hydroxy 3-quinoléine carboxylate d'éthyle, porte au reflux pendant 16 heures, évapore à sec sous pression réduite, reprend dans 100 ml d'une solution aqueuse normale d'acide chlorhydrique, chauffe 2 heures à 70 °C et traite au charbon actif. Le filtrat cristallise, on essore, lave avec une solution aqueuse normale d'acide chlorhydrique, sèche sous pression réduite, recristallise dans l'acide acétique, et récupère 2,2 g de produit attendu. F > 260 °C.

Analyse : $C_{15}H_{16}N_3O_2SCl$ = 337,83

| | | | | | |
|---|---|---|---|---|---|
| Calculé : | C % 53,33 | H % 4,77 | N % 12,44 | S % 9,49 | Cl % 10,49 |
| Trouvé : | C % 53,1 | H % 4,7 | N % 12,3 | S % 9,5 | Cl % 10,3 |

En opérant selon un procédé analogue à celui décrit ci-dessus à l'exemple 2, on a préparé les produits des exemples 3 à 12 ci-après.

(Voir Tableaux pages suivantes)

Tableau n° 1

| Exemple n° | 3 | 4 | 5 | 6 |
|---|---|---|---|---|
| Produit | Chlorhydrate de N-(4,5-dihydrothiazol-2-yl) 4-hydroxy 6-méthyl 3-quinoléine carboxamide | Chlorhydrate de N-(4,5-dihydrothiazol-2-yl) 4-hydroxy 6-isopropyl 3-quinoléine carboxamide | Chlorhydrate de N-(4,5-dihydrothiazol-2-yl) 4-hydroxy 7-chloro 3-quinoléine carboxamide | Chlorhydrate de N-(4,5-dihydrothiazol-2-yl) 4-hydroxy 6-chloro 3-quinoléine carboxamide |
| R................. | H | H | H | H |
| $R_1$ ............. | 6-$CH_3$ | 6-$CH(CH_3)_2$ | 7-Cl | 6-Cl |
| Particularités . | 48 H de reflux | 48 H de reflux | 48 H de reflux | 48 H de reflux |
| Solvant de cristallisation .... | Acide acétique | Acide acétique | Acide acétique | Acide acétique |
| P F en ° C ..... | > 260 | > 260 | > 260 | > 260 |
| Formule brute .. | $C_{14}H_{14}ClN_3O_2S$ | $C_{16}H_{18}ClN_3O_2S$ | $C_{13}H_{11}Cl_2N_3O_2S$ | $C_{13}H_{11}Cl_2N_3O_2S$ |
| P M ........... | 323,803 | 351,85 | 344,22 | 344,22 |
| Microanalyse ... Calculé/trouvé | | | | |
| C % | 51,93 / 51,6 | 54,62 / 54,6 | 45,36 / 45,2 | 45,36 / 45,1 |
| H % | 4,35 / 4,5 | 5,15 / 5,1 | 3,22 / 3,3 | 3,22 / 3,3 |
| N % | 12,97 / 12,9 | 11,94 / 11,7 | 12,20 / 12,3 | 12,20 / 12,3 |
| Cl % | 10,94 / 10,9 | 10,07 / 10,4 | 20,60 / 20,6 | 20,60 / 20,3 |
| S % | 9,90 / 9,7 | 9,11 / 9,0 | 9,31 / 9,0 | 9,31 / 9,5 |

Tableau n° 2

| Exemple n° | 7 | 8 | 9 | 10 |
|---|---|---|---|---|
| Produit | Chlorhydrate de N-(4,5-dihydrothiazol-2-yl) 4-hydroxy 6-fluoro 3-quinoléine carboxamide | Chlorhydrade de N-(4,5-dihydrothiazol-2-yl) 4-hydroxy 7-méthoxy 3-quinoléine carboxamide | Chlorhydrate de N-(4,5-dihydrothiazol-2-yl) 4-hydroxy 2-méthyl 3-quinoléine carboxamide | Chlorhydrate de N-(4,5-dihydrothiazol-2-yl) 4-hydroxy 6-méthylthio 3-quinoléine carboxamide |
| R .............. | H | H | $CH_3$ | H |
| $R_1$ ............ | 6-F | 7-$OCH_3$ | H | 6-$SCH_3$ |
| Particularités . | 48 H de reflux | 48 H de reflux | 48 H de reflux | 48 H de reflux |
| Solvant de cristallisation .... | Acide acétique | Acide acétique | Acide acétique | Acide acétique |
| P F en ° C ..... | > 260 | > 260 | 280 avec décomposition | > 260 |
| Formule brute .. | $C_{13}H_{11}ClFN_3O_2S$ | $C_{14}H_{14}ClN_3O_3S$ | $C_{14}H_{14}ClN_3O_2S$ | $C_{14}H_{14}ClN_3O_2S_2$ |
| P M ........... | 327,76 | 339,79 | 323,803 | 355,80 |

| Microanalyse ... Calculé/trouvé | 7 calculé | 7 trouvé | 8 calculé | 8 trouvé | 9 calculé | 9 trouvé | 10 calculé | 10 trouvé |
|---|---|---|---|---|---|---|---|---|
| C % | 47,64 | 47,4 | 49,50 | 49,8 | 51,92 | 52,2 | 47,26 | 47,2 |
| H % | 3,38 | 3,4 | 4,15 | 4,1 | 4,36 | 4,5 | 3,96 | 4,0 |
| N % | 12,82 | 12,7 | 12,36 | 12,2 | 12,97 | 13,3 | 11,81 | 11,5 |
| Cl % | 10,81 | 10,4 | 10,43 | 10,1 | 10,95 | 10,6 | 9,96 | 9,7 |
| S % | 9,78 | 9,7 | 9,43 | 9,2 | 9,90 | 9,9 | 18,02 | 17,8 |
| F % | 5,79 | 5,9 | | | | | | |

Tableau n° 3

| Exemple n° | 11 | | 12 | |
|---|---|---|---|---|
| Produit | Chlorhydrate de N-(4,5-dihydrothiazol-2-yl) 4-hydroxy 6-trifluorométhyl 3-quinoléine carboxamide | | Chlorhydrate de N-(4,5-dihydrothiazol-2-yl) 4-hydroxy 6-méthoxy 3-quinoléine carboxamide | |
| R ............. | H | | H | |
| $R_1$ ............. | 6-$CF_3$ | | 6-$OCH_3$ | |
| Particularités . | 48 H de reflux | | 48 H de reflux | |
| Solvant de cristallisation .... | Acide acétique | | Acide acétique | |
| P F en ° C ..... | > 260 | | > 260 | |
| Formule brute .. | $C_{14}H_{11}ClF_3N_3O_2S$ | | $C_{14}H_{14}ClN_3O_3S$ | |
| P M ........... | 377,77 | | 339,77 | |
| Microanalyse ... Calculé/trouvé | | | | |
| C % | 44,51 | 44,3 | 49,49 | 49,5 |
| H % | 2,93 | 2,9 | 4,15 | 4,1 |
| N % | 11,12 | 11,1 | 12,36 | 12,3 |
| Cl % | 9,38 | 9,4 | 10,43 | 10,7 |
| S % | 8,48 | 8,7 | 9,43 | 9,4 |
| F % | 15,08 | 15,3 | | |

Le 4-hydroxy 6-éthyl 3-quinoléine carboxylate d'éthyle de départ de l'exemple 2 est obtenu comme suit :

Stade A

4-éthyl phénylaminométhylène propanedioate d'éthyle.

On mélange sous agitation et sous courant de gaz inerte 50 g de 4-éthyl aniline et 89 g d'éthoxyméthylènemalonate d'éthyle. On porte le mélange à 160 °C en distillant l'éthanol formé. Par refroidissement, on obtient le 4-éthyl phénylaminométhylène propanedioate d'éthyle attendu sous forme d'huile que l'on utilise telle quelle dans le stade suivant.

Stade B

4-hydroxy 6-éthyl 3-quinoléine carboxylate d'éthyle.

On mélange sous agitation et sous courant de gaz inerte le produit préparé au stade A, avec 70 $cm^3$ d'oxyde de phényle. On chauffe le mélange obtenu à 250 °C pendant 1 heure en distillant l'éthanol formé. On refroidit le mélange, ajoute 30 $cm^3$ d'acétone. On obtient ainsi un précipité que l'on essore, empâte avec 30 $cm^3$ d'acétone et que l'on sèche. On recristallise dans l'éthanol et obtient ainsi 36 g de 4-hydroxy 6-éthyl 3-quinoléine carboxylate d'éthyle. F > 260 °C.

Microanalyse : $C_{14}H_{15}NO_3$ : 245,28

Calculé : C % 68,55 H % 6,16 N % 5,71

Trouvé : C % 68,6 H % 6,1 N % 5,7

En opérant selon un procédé analogue à celui ci-dessus décrit, on a préparé les produits de formule II'

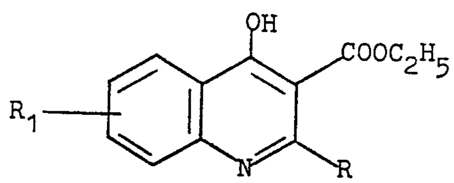

(II')

suivants :

(Voir Tableaux pages suivantes)

| Produit pour l'exemple n° | 3 | 6 | 7 | 8 |
|---|---|---|---|---|
| R ................ | H | H | H | H |
| $R_1$ ............... | 6-$CH_3$ | 6-Cl | 6-F | 7-$OCH_3$ |
| Produit de départ | 4-méthylaniline | 4-chloroaniline | 4-fluoroaniline | 3-méthoxyaniline |
| P F en ° C ....... | > 260 | > 260 | > 260 | > 260 |
| Formule brute .... | $C_{13}H_{13}NO_3$ | $C_{12}H_{10}ClNO_3$ | $C_{12}H_{10}FNO_3$ | $C_{13}H_{13}NO_4$ |
| P M .............. | 231,24 | 251,7 | 234,22 | 247,31 |
| Microanalyse Calculé/trouvé ... | | | | |
| C % | 67,52 / 67,2 | 57,27 / 57,5 | 61,53 / 61,3 | 63,15 / 63,1 |
| H % | 5,69 / 5,7 | 4,00 / 4,0 | 4,30 / 4,3 | 5,3 / 5,4 |
| N % | 6,05 / 6,0 | 5,56 / 5,5 | 5,93 / 6,0 | 5,66 / 5,7 |
| Cl % | | 14,08 / 14,0 | | |
| F % | | | 8,11 / 8,0 | |

| Produit pour l'exemple n° | 10 | | 11 | | 12 | |
|---|---|---|---|---|---|---|
| R | H | | H | | H | |
| $R_1$ | 6-$SCH_3$ | | 6-$CF_3$ | | 6-$OCH_3$ | |
| Produit de départ | 4-méthylthioaniline | | 4-trifluorométhylaniline | | 4-méthoxyaniline | |
| P F en ° C | > 260 | | > 260 | | > 260 | |
| Formule brute | $C_{13}H_{13}NO_3S$ | | $C_{13}H_{10}NO_3F_3$ | | $C_{13}H_{13}NO_4$ | |
| P M | 263,31 | | 285,00 | | 247,31 | |
| Microanalyse Calculé/trouvé | | | | | | |
| C % | 59,3 | 59,0 | 54,78 | 54,5 | 63,15 | 63,1 |
| H % | 4,97 | 5,0 | 3,53 | 3,5 | 5,3 | 5,4 |
| N % | 5,32 | 5,4 | 4,91 | 4,9 | 5,66 | 5,7 |
| F % | | | 19,99 | 20,2 | | |
| S % | 12,17 | 12,3 | | | | |

Exemple 13

On a préparé des comprimés répondant à la formule :
— Chlorhydrate de N-(4,5-dihydrothiazol-2-yl) 4-hydroxy 3-quinoléine carboxamide 5 mg
— Excipient q.s. pour un comprimé terminé à 100 mg
(détail de l'excipient : lactose, amidon, talc, stéarate de magnésium).

Exemple 14

On a préparé des comprimés répondant à la formule :
— Chlorhydrate de N-(4,5-dihydrothiazol-2-yl) 6-éthyl-4-hydroxy-3-quinoléine carboxamide 5 mg
— Excipient q.s. pour un comprimé terminé à 100 mg
(détail de l'excipient : lactose, amidon, talc, stéarate de magnésium).

Exemple 15

On a préparé des comprimés répondant à la formule :
— Chlorhydrate de N-(4,5-dihydrothiazol-2-yl) 6-chloro-4 hydroxy-3-quinoléine carboxamide 10 mg
— Excipient q.s. pour un comprimé terminé à 100 mg
(détail de l'excipient ; lactose, amidon, talc, stéarate de magnésium).

Etude pharmacologique :

1. Affinité pour les récepteurs des benzodiazépines

La technique est inspirée de celle de Möhler et Okada : Science n° 198 p. 849-851 (1977).

On homogéinise au vingtième (poids/volume) dans le sucrose 0,32 M, les cortex prélevés sur des cerveaux de rats mâles pesant 150 g en moyenne. Après centrifugation à 1 000 g du mélange homogéinisé pendant 10 minutes à 0 °C, le surnageant est centrifugé à 30 000 g pendant 20 minutes à + 4 °C. Le culot est mis en suspension dans 20 volumes de tampon Tris HCl 50 mM pH 7,4, et centrifugé à 30 000 g pendant 20 minutes à + 4 °C. Le nouveau culot obtenu est mis en suspension dans 50 ml de tampon Krebs Tris HCl pH 7,4.

On fait ensuite incuber pendant 30 minutes à 0 °C, 2 ml de suspension en présence de $^3H$ diazepam à la concentration $10^{-9}$ M, seul, avec des concentrations croissantes du produit à tester, ou, pour déterminer la fixation non spécifique, avec du diazepam non radioactif à la concentration $10^{-6}$ M.

Les suspensions incubées sont filtrées sur Whatman GF/C et les filtres sont lavés par deux fois 5 ml de tampon Krebs Tris HCl pH 7,4 à 0 °C.

La radioactivité des filtres est mesurée par scintillation liquide.

L'activité du produit est exprimée en C.I. 50 : concentration inhibant 50 % de la liaison spécifique du $^3H$ Diazepam déterminé graphiquement.

Résultats :

| Produit de l'exemple | CI 50 en nM |
|---|---|
| 1 | 0,5 |
| 2 | 0,3 |
| 3 | 1,5 |
| 4 | 2,2 |
| 5 | 0,9 |
| 6 | 1,0 |
| 7 | 1,3 |
| 8 | 1,2 |
| 9 | 2,6 |

2. Test des 4 plaques (activité anxiolytique)

Technique :

L'appareil (Apelab) et le protocole utilisé ont été décrits par Boissier et Coll. (European J. Pharmacol. 1968, *4*, 145). Les plaques sont reliées à un stimulateur (U. Sachs, Roucaire) qui permet de délivrer des chocs électriques de 120 volts pendant 0,5 s. Les essais sont réalisés sur des lots de 10 souris 1/2 heure

après administration per os du composé étudié. Chaque animal est déposé individuellement sur l'appareil. Après 15 secondes d'exploration libre, il est soumis à un choc électrique chaque fois qu'il passe d'une plaque à l'autre, un minimum de 3 secondes étant observé entre deux chocs. Le nombre de chocs délivrés est compté pendant 1 minute. Les résultats obtenus sont comparés à ceux observés chez des animaux témoins par un test de Dunnett.

On a indiqué ci-après la dose orale administrée pour laquelle le nombre de chocs délivrés est augmenté de façon maximale.

| Produit de l'exemple | Dose mg/Kg |
|---|---|
| 1 | 10 |
| 2 | 20 |
| 4 | 20 |
| 6 | 50 |
| 8 | 20 |
| 9 | 10 |

Les produits des exemples 1, 2, 4, 6, 8 et 9 présentent donc une bonne activité anxiolytique.

3. Action sur stress au bruit révélé par le taux de corticostérone plasmatique (activité anxiolytique).

Technique :

Les essais sont effectués sur des lots de 5 rats. Le composé étudié est administré per os entre 8 et 9 heures du matin, les animaux étant groupés par 5 depuis 24 heures.

Une heure après, le stress est appliqué ; il consiste à changer les animaux de cage, à les amener dans une pièce où un poste de radio fonctionne bruyamment puis, 1/2 heure plus tard, à leur faire une piqûre intrapéritonéale. Dix minutes après celle-ci, les rats sont anesthésiés à l'halothane et environ 3 ml de sang sont prélevés après décapitation.

Les taux sériques de corticostérone sont déterminés par une méthode radio-immunologique. Compte tenu de l'importance, chez le rat mâle, des concentrations sériques de corticostérone, le dosage est réalisé directement, sans extraction préalable de l'hormone, sur 0,1 ml de l'échantillon dilué au 1/100. A la prise d'essai sont ajoutés dans des tubes à hémolyse (12 × 65 mm) 0,1 ml d'une solution aqueuse de $^3$H-corticostérone (5 000 cpm) et 0,5 ml de tampon phosphate (0,02 M ; pH 6,9) additionné de 0,02 % de gélatine et 0,02 % de nitrure de sodium, renfermant en suspension l'antisérum anticorticostérone (dilution 1/300 000), l'antisérum de mouton anti $\gamma$-globuline de lapin (dilution 1/50) et du sérum normal de lapin traité au charbon Dextran (dilution 1/500).

Le volume est ajusté à 1 ml et l'incubation réalisée en une nuit à 4 °C. Après centrifugation (3 000 t/mn) pendant 20 mn, le surnageant est éliminé et la radioactivité contenue dans le précipité est mesurée dans un compteur, après addition de 2 ml de liquide de scintillation renfermant 5 % de Soluène 350. Les concentrations de corticostérone sont déterminées au moyen d'une courbe d'étalonnage (0-4 000 pg) établie dans les conditions du traitement des échantillons.

Le taux sérique individuel de corticostérone, exprimé en µg/100 ml, est la moyenne de trois déterminations. Les résultats sont ensuite comparés par le test de Student à ceux obtenus chez les animaux témoins ayant subi le même stress.

Les résultats sont exprimés en DE 50 (Dose Efficace 50), c'est-à-dire la dose administrée per os réduisant de 50 % le taux de corticostérone des animaux traités.

Résultats :

| Produits de l'exemple | DE 50 mg/Kg |
|---|---|
| 1 | 10 |
| 2 | 15 |
| 4 | 50 |
| 6 | 50 |
| 9 | 10 |

Le stress est moins important chez les animaux traités ; ces animaux ont donc un degré d'anxiété moindre que celui des témoins.

4. Etude de la toxicité aiguë.

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produits de l'exemple | $DL_0$ en mg/kg |
|---|---|
| 1 | $>400$ |
| 2 | $>400$ |
| 4 | $>1000$ |
| 5 | $>1000$ |
| 6 | $>400$ |
| 8 | $>400$ |
| 9 | $>1000$ |

**Revendications** (pour les Etats contractants : BE, CH, DE, GB, IT, LI, LU, NL, SE)

1. Les dérivés du N-dihydrothiazolyl-3-quinoléine carboxamide, ainsi que leurs sels d'addition avec les acides minéraux ou organiques, caractérisés en ce qu'ils répondent à la formule générale I

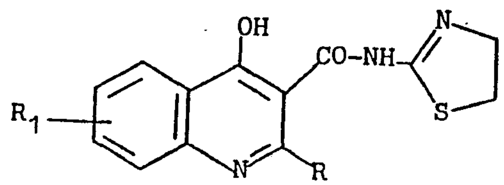

(I)

dans laquelle R représente un atome d'hydrogène ou un radical alcoyle renfermant de 1 à 4 atomes de carbone, et $R_1$ en position 6 ou 7, représente un atome d'hydrogène ou d'halogène, un radical alcoyle linéaire renfermant de 1 à 4 atomes de carbone, un radical alcoyle ramifié renfermant de 3 à 5 atomes de carbone, un radical alcoxy renfermant de 1 à 4 atomes de carbone, un radical trifluorométhylthio, méthylthio, trifluorométhoxy ou trifluorométhyle.

2. Les dérivés du N-dihydrothiazolyl-3-quinoléine carboxamide tels que définis par la formule I de la revendication 1, ainsi que leurs sels d'addition avec les acides minéraux ou organiques, caractérisés en ce que R représente un atome d'hydrogène ou un radical méthyle, $R_1$ représente un atome d'hydrogène ou de chlore ou un radical méthoxy, ou $R_1$ en position 6 représente un atome de fluor, ou un radical méthyle, éthyle, isopropyle, méthylthio ou trifluorométhyle.

3. Les dérivés du N-dihydrothiazolyl-3-quinoléine carboxamide selon la revendication 2, ainsi que leurs sels d'addition avec les acides minéraux ou organiques, caractérisés en ce que R représente un atome d'hydrogène.

4. Le N-(4,5-dihydrothiazol-2-yl) 4-hydroxy 3-quinoléine carboxamide ainsi que ses sels d'addition avec les acides minéraux ou organiques.

5. Le N-(4,5-dihydrothiazol-2-yl) 6-éthyl 4-hydroxy 3-quinoléine carboxamide ainsi que ses sels d'addition avec les acides minéraux ou organiques.

6. Le N-(4,5-dihydrothiazol-2-yl) 6-chloro 4-hydroxy 3-quinoléine carboxamide ainsi que ses sels d'addition avec les acides minéraux ou organiques.

7. Le N-(4,5-dihydrothiazol-2-yl) 4-hydroxy 2-méthyl 3-quinoléine carboxamide ainsi que ses sels d'addition avec les acides minéraux ou organiques.

8. Procédé de préparation des dérivés tels que définis par la formule I de la revendication 1, ainsi que de leurs sels, caractérisé en ce que l'on fait réagir un produit de formule II

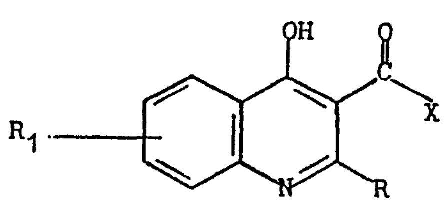

(II)

dans laquelle R et $R_1$ ont la signification déjà indiquée, et X représente un atome de chlore ou de brome, ou un radical hydroxy ou alcoxy renfermant de 1 à 5 atomes de carbone avec la 2-aminothiazoline, pour obtenir un produit de formule (I) :

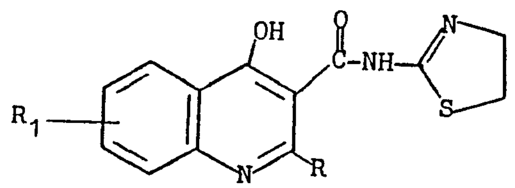

(I)

dans laquelle R et $R_1$ ont la signification déjà indiquée, que l'on isole et salifie si désiré.

9. Procédé selon la revendication 8, caractérisé en ce que l'on utilise au départ un produit de formule (II) dans laquelle X représente un atome de chlore ou un radical éthoxy.

10. Médicaments caractérisés en ce qu'ils sont constitués par les dérivés de la N-dihydrothiazolyl-3-quinoléine carboxamide tels que définis par la formule I de la revendication 1, ainsi que par leurs sels d'addition avec les acides pharmaceutiquement acceptables.

11. Médicaments caractérisés en ce qu'ils sont constitués par les dérivés de la N-dihydrothiazolyl-3-quinoléine carboxamide tels que définis dans l'une des revendications 2 ou 3, ainsi que par leurs sels d'addition avec les acides pharmaceutiquement acceptables.

12. Médicaments caractérisés en ce qu'ils sont constitués par les dérivés de la N-dihydrothiazolyl-3-quinoléine carboxamide, tels que définis dans l'une des revendications 4, 5, 6 ou 7 ainsi que par leurs sels d'addition avec les acides pharmaceutiquement acceptables.

13. Compositions pharmaceutiques caractérisées en ce qu'elles renferment à titre de principe actif, l'un au moins des médicaments définis à l'une quelconque des revendications 10 à 12.

**Revendications** (pour l'Etat contractant AT)

1. Procédé pour préparer les dérivés du N-dihydrothiazolyl 3-quinoléine carboxamide, ainsi que leurs sels d'addition avec les acides minéraux ou organiques répondant à la formule générale (I) :

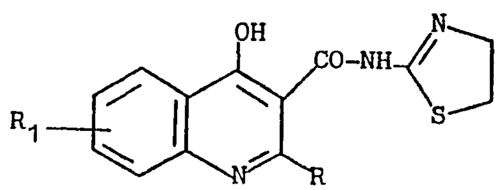

(I)

dans laquelle R représente un atome d'hydrogène ou un radical alcoyle renfermant de 1 à 4 atomes de carbone, et $R_1$ en position 6 ou 7, représente un atome d'hydrogène ou d'halogène, un radical alcoyle linéaire renfermant de 1 à 4 atomes de carbone, un radical alcoyle ramifié renfermant de 3 à 5 atomes de carbone, un radical alcoxy renfermant de 1 à 4 atomes de carbone, un radical trifluorométhylthio, méthylthio, trifluorométhoxy ou trifluorométhyle, caractérisé en ce que l'on fait réagir un produit de formule (II)

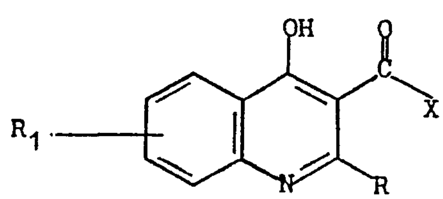

(II)

dans laquelle R et $R_1$ ont la signification déjà indiquée, et X représente un atome de chlore ou de brome, ou un radical hydroxy ou alcoxy renfermant de 1 à 5 atomes de carbone avec la 2-aminothiazoline, pour obtenir un produit de formule (I) :

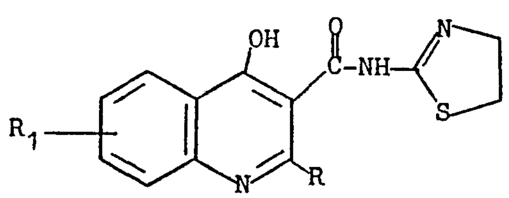

(I)

dans laquelle R et $R_1$ ont la signification déjà indiquée, que l'on isole et salifie si désiré.

2. Procédé selon la revendication 1, caractérisé en ce que l'on utilise au départ un produit de formule (II) dans laquelle X représente un atome de chlore ou de brome et que l'on opère en présence d'un agent fixateur d'hydracide.

3. Procédé selon la revendication 1, caractérisé en ce que l'on utilise au départ un produit de formule (II) dans laquelle X représente un radical alcoxy renfermant de 1 à 5 atomes de carbone et que l'on opère en présence d'un dérivé alcoylé de l'aluminium.

4. Procédé selon l'une quelconque des revendications 1 à 3, caractérisé en ce que l'on utilise au départ un produit de formule (II) dans laquelle R représente un atome d'hydrogène ou un radical méthyle, $R_1$ représente un atome d'hydrogène ou de chlore ou un radical méthoxy, ou $R_1$ en position 6, représente un atome de fluor, ou un radical méthyle, éthyle, isopropyle, méthylthio, ou trifluorométhyle.

5. Procédé selon la revendication 4, caractérisé en ce que l'on utilise au départ un produit de formule (II) dans laquelle R représente un atome d'hydrogène.

6. Procédé selon l'une quelconque des revendications 1 à 5, caractérisé en ce que l'on utilise au départ un produit de formule (II) dans laquelle R et $R_1$ représentent un atome d'hydrogène.

7. Procédé selon l'une quelconque des revendications 1 à 5, caractérisé en ce que l'on utilise au départ un produit de formule (II) dans laquelle R représente un atome d'hydrogène et $R_1$ représente un radical éthyle en position 6.

8. Procédé selon l'une quelconque des revendications 1 à 5, caractérisé en ce que l'on utilise au départ un produit de formule (II) dans laquelle R représente un atome d'hydrogène et $R_1$ représente un atome de chlore en position 6.

9. Procédé selon l'une quelconque des revendications 1 à 5, caractérisé en ce que l'on utilise au départ un produit de formule (II) dans laquelle R représente un radical méthyle et $R_1$ représente un atome d'hydrogène.

**Claims** (for the Contracting States : BE, CH, DE, GB, IT, LI, LU, NL, SE)

1. Derivatives of N-dihydrothiazolyl-3-quinoline carboxamide, as well as their salts of addition with mineral or organic acids, characterized in that they answer to the general formula (I) :

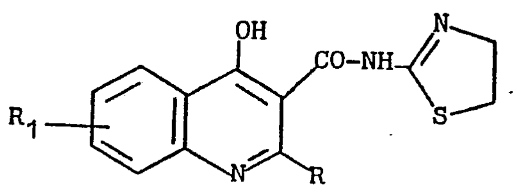

(I)

in which R represents a hydrogen atom or an alkyl radical containing from 1 to 4 carbon atoms and $R_1$ in position 6 or 7, represents a hydrogen or halogen atom, a linear alkyl radical containing from 1 to 4 carbon atoms, a branched alkyl radical containing from 3 to 5 carbon atoms, an alkoxy radical containing from 1 to 4 carbon atoms, a trifluoromethylthio, methylthio, trifluoromethoxy, or trifluoromethyl radical.

2. Derivatives of N-dihydrothiazolyl-3-quinoline carboxamide as defined by formula (I) of Claim 1, as well as their salts of addition with mineral or organic acids, characterized in that R represents a hydrogen atom or a methyl radical, $R_1$ represents a hydrogen or a chlorine atom or a methoxy radical, or $R_1$ in position 6 represents a fluorine atom, or a methyl, ethyl, isopropyl, methylthio or trifluoromethyl radical.

3. Derivatives of N-dihydrothiazolyl-3-quinoline carboxamide according to Claim 2, as well as their salts of addition with mineral or organic acids, characterized in that R represents a hydrogen atom.

4. N-(4,5-dihydrothiazol-2-yl)-4-hydroxy-3-quinoline carboxamide, as well as its salts of addition with mineral or organic acids.

5. N-(4,5-dihydrothiazol-2-yl)-6-chloro-4-hydroxy-3-quinoline carboxamide, as well as its salts of addition with mineral or organic acids.

6. N-(4,5-dihydrothiazol-2-yl)-6-chloro-4-hydroxy-3-quinoline carboxamide, as well as its salts of addition with mineral or organic acids.

7. N-(4,5-dihydrothiazol-2-yl)-4-hydroxy-2-methyl-3-quinoline carboxamide, as well as its salts of addition with mineral or organic acids.

8. Process for the preparation of the derivatives as defined by formula (I) of Claim 1, as well as their salts, characterized in that a product with the formula (II) :

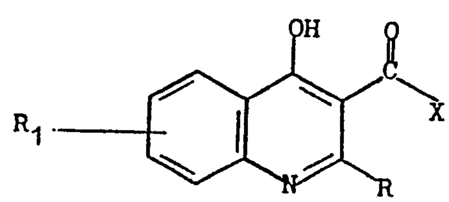

(II)

in which R and $R_1$ have the significance already indicated, and X represents an atom of chlorine or bromine, or a hydroxy or alkoxy radical containing from 1 to 5 carbon atoms, is made to react with 2-aminothiazoline, in order to obtain a product with the formula (I) :

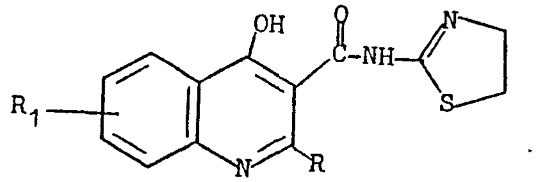

(I)

in which R and $R_1$ have the significance already indicated, which, if desired, is isolated and salified.

9. Process according to Claim 8, characterized in that there is used at the start a product with the formula (II) in which X represents a chlorine atom or an ethoxy radical.

10. Medicaments, characterized in that they are constituted by the derivatives of N-dihydrothiazolyl-3-quinoline carboxamide as defined by formula I of Claim 1, as well as by their salts of addition with pharmaceutically acceptable acids.

11. Medicaments, characterized in that they are constituted by the derivatives of N-dihydrothiazolyl-3-quinoline carboxamide as defined in one of the Claims 2 or 3, as well as by their salts of addition with pharmaceutically acceptable acids.

12. Medicaments, characterized in that they are constituted by the derivatives of N-dihydrothiazolyl-3-quinoline carboxamide, as defined in one of the Claims 4, 5, 6 or 7 as well as by their salts of addition with pharmaceutically acceptable acids.

13. Pharmaceutical compositions characterized in that they contain as active principle, one at least of the medicaments defined in any one of the Claims 10 to 12.

**Claims** (for the Contracting State AT)

1. Process for preparing the derivatives of N-dihydrothiazolyl-3-quinoline carboxamide, as well as their salts of addition with mineral or organic acids, answering to the general formula (I) :

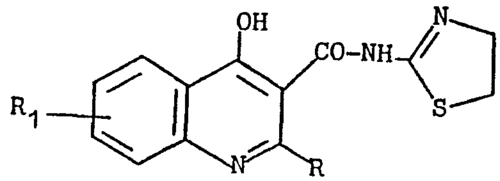

(I)

in which R represents a hydrogen atom or an alkyl radical containing from 1 to 4 carbon atoms and $R_1$, in position 6 or 7, represents a hydrogen or halogen atom, a linear alkyl radical containing from 1 to 4 carbon atoms, a branched alkyl radical containing from 3 to 5 carbon atoms, an alkoxy radical containing from 1 to 4 carbon atoms, a trifluoromethylthio, methylthio, trifluoromethoxy or trifluoromethyl radical, characterised in that a product with the formula (II)

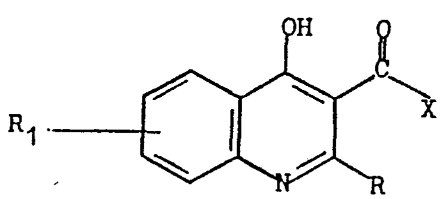

(II)

in which R and $R_1$ have the significance already indicated, and X represents a chlorine or a bromine atom, or a hydroxy or an alkoxy radical containing from 1 to 5 carbon atoms, is made to react with 2-aminothiazoline, so as to obtain a product with the formula (I)

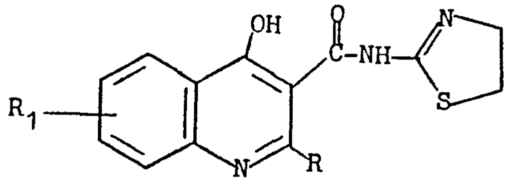

(I)

in which R and $R_1$ have the significance already indicated, which is isolated and salified if desired.

2. Process according to Claim 1, characterized in that, at the start, a product with the formula (II) is utilized in which X represents a chlorine or a bromine atom and that the operation is carried out in the presence of a hydracid fixing agent.

3. Process according to Claim 1, characterized in that, at the start, a product with the formula (II) is utilized, in which X represents an alkoxy radical containing from 1 to 5 carbon atoms, and that the operation is carried out in the presence of an alkyl derivative of aluminium.

4. Process according to any one of Claims 1 to 3, characterized in that, at the start, a product with the formula (II) is utilized in which R represents a hydrogen atom or a methyl radical, $R_1$ represents a hydrogen or a chlorine atom or a methoxy radical, or $R_1$, in position 6, represents a fluorine atom, or a methyl, ethyl, isopropyl, methylthio or trifluoromethyl radical.

5. Process according to Claim 4, characterized in that, at the start, a product with the formula (II) is utilized in which R represents a hydrogen atom.

6. Process according to any one of the Claims 1 to 5, characterized in that, at the start, a product with the formula (II) is utilized in which each of R and $R_1$ represents a hydrogen atom.

7. Process according to any one of Claims 1 to 5, characterized in that, at the start, a product with the formula (II) is utilized in which each of R represents a hydrogen atom and $R_1$ represents an ethyl radical in position 6.

8. Process according to any one of the Claims 1 to 5, characterized in that, at the start, a product with the formula (II) is utilized in which R represents a hydrogen atom and $R_1$ represents a chlorine atom in position 6.

9. Process according to any one of the Claims 1 to 5, characterized in that, at the start, a product with the formula (II) is utilized in which R represents a methyl radical and $R_1$ represents a hydrogen atom.

**Ansprüche** (für die Vertragsstaaten : BE, CH, DE, GB, IT, LI, LU, NL, SE)

1. N-Dihydrothiazolyl-3-chinolin-carboxamid-Derivate sowie deren Additionssalze mit Mineral- oder organischen Säuren, dadurch gekennzeichnet, daß sie der allgemeinen Formel I

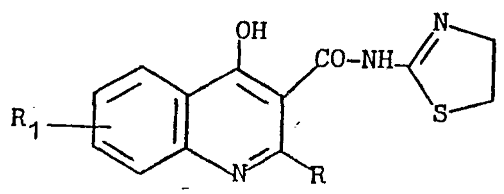

(I)

entsprechen, worin R ein Wasserstoffatom oder einen Alkylrest mit 1 bis 4 Kohlenstoffatomen bedeutet und $R_1$ in 6- oder 7-Stellung ein Wasserstoff- oder Halogenatom, einen linearen Alkylrest mit 1 bis 4 Kohlenstoffatomen, einen verzweigten Alkylrest mit 3 bis 5 Kohlenstoffatomen, einen Alkoxyrest mit 1 bis 4 Kohlenstoffatomen, einen Trifluormethylthio-, Methylthio-, Trifluormethoxy- oder Trifluormethylrest bedeutet.

2. N-Dihydrothiazolyl-3-chinolin-carboxamid-Derivate der Formel I gemäß Anspruch 1 sowie deren Additionssalze mit Mineral- oder organischen Säuren, dadurch gekennzeichnet, daß R ein Wasserstoffatom oder einen Methylrest bedeutet, $R_1$ ein Wasserstoffatom oder Chloratom oder einen Methoxyrest darstellt oder $R_1$ in 6-Stellung ein Fluoratom oder einen Methyl-, Ethyl-, Isopropyl-, Methylthio- oder Trifluormethylrest bedeutet.

3. N-Dihydrothiazolyl-3-chinolin-carboxamid-Derivate gemäß Anspruch 2 sowie deren Additionssalze mit Mineral- oder organischen Säuren, dadurch gekennzeichnet, daß R ein Wasserstoffatom bedeutet.

4. N-(4,5-Dihydrothiazol-2-yl)-4-hydroxy-3-chinolin-carboxamid sowie dessen Additionssalze mit Mineral- oder organischen Säuren.

5. N-(4,5-Dihydrothiazol-2-yl)-6-ethyl-4-hydroxy-3-chinolin-carboxamid sowie dessen Additionssalze mit Mineral- oder organischen Säuren.

6. N-(4,5-Dihydrothiazol-2-yl)-6-chlor-4-hydroxy-3-chinolin-carboxamid sowie dessen Additionssalze mit Mineral- oder organischen Säuren.

7. N-(4,5-Dihydrothiazol-2-yl)-4-hydroxy-2-methyl-3-chinolin-carboxamid sowie dessen Additionssalze mit Mineral- oder organischen Säuren.

8. Verfahren zur Herstellung der Derivate der Formel I gemäß Anspruch 1 sowie von deren Salzen, dadurch gekennzeichnet, daß man ein Produkt der Formel II

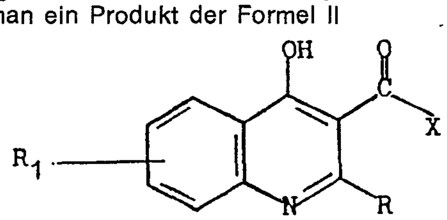

(II)

worin R und $R_1$ die angegebene Bedeutung besitzen und X ein Chlor- oder Bromatom oder einen Hydroxyrest oder eine Alkoxygruppe mit 1 bis 5 Kohlenstoffatomen bedeutet, mit 2-Aminothiazolin umsetzt, um ein Produkt der Formel (I)

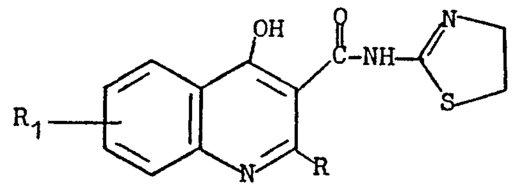

(I)

zu erhalten, worin R und $R_1$ die angegebene Bedeutung besitzen, das man isoliert und gewünschtenfalls in ein Salz überführt.

9. Verfahren gemäß Anspruch 8, dadurch gekennzeichnet, daß man von einem Produkt der Formel (II) ausgeht, worin X ein Chloratom oder einen Ethoxyrest bedeutet.

10. Arzneimittel, dadurch gekennzeichnet, daß sie aus den N-Dihydrothiazolyl-3-chinolin-carboxamid-Derivaten der Formel I gemäß Anspruch 1 sowie aus deren Additionssalzen mit pharmazeutisch verträglichen Säuren bestehen.

11. Arzneimittel, dadurch gekennzeichnet, daß sie aus den N-Dihydrothiazolyl-3-chinolin-carboxamid-Derivaten gemäß einem der Ansprüche 2 oder 3 sowie aus deren Additionssalzen mit pharmazeutisch verträglichen Säuren bestehen.

12. Arzneimittel, dadurch gekennzeichnet, daß sie aus den N-Dihydrothiazolyl-3-chinolin-carboxamid-Derivaten gemäß einem der Ansprüche 4, 5, 6 oder 7 sowie aus deren Additionssalzen mit pharmazeutisch verträglichen Säuren bestehen.

13. Pharmazeutische Zusammensetzungen, dadurch gekennzeichnet, daß sie als Wirkstoff zumindest eines der gemäß einem der Ansprüche 10 bis 12 definierten Arzneimittel enthalten.

**Ansprüche** (für den Vertragsstaat AT)

1. Verfahren zur Herstellung von N-Dihydrothiazolyl-3-chinolin-carboxamid-Derivaten sowie von deren Additionssalzen mit Mineral- oder organischen Säuren der allgemeinen Formel (I)

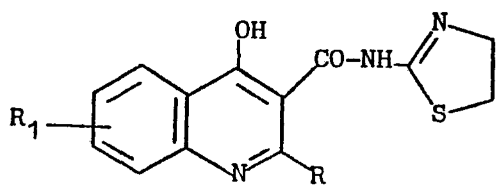

(I)

worin R ein Wasserstoffatom oder einen Alkylrest mit 1 bis 4 Kohlenstoffatomen bedeutet und $R_1$ in 6- oder 7-Stellung ein Wasserstoff- oder Halogenatom, einen linearen Alkylrest mit 1 bis 4 Kohlenstoffatomen, einen verzweigten Alkylrest mit 3 bis 5 Kohlenstoffatomen, einen Alkoxyrest mit 1 bis 4 Kohlenstoffatomen, einen Trifluormethylthio-, Methylthio-, Trifluormethoxy- oder Trifluormethylrest bedeutet, dadurch gekennzeichnet, daß man ein Produkt der Formel (II)

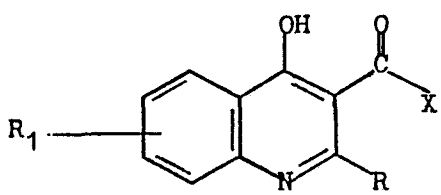

(II)

worin R und $R_1$ die vorstehend angegebene Bedeutung besitzen und X ein Chlor- oder Bromatom oder einen Hydroxyrest oder eine Alkoxygruppe mit 1 bis 5 Kohlenstoffatomen bedeutet, mit 2-Aminothiazolin umsetzt, um ein Produkt der Formel (I)

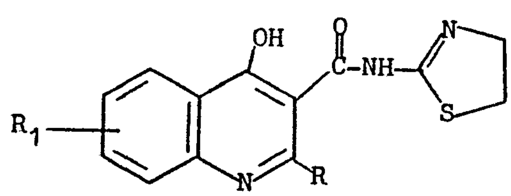

(I)

zu erhalten, worin R und $R_1$ die angegebene Bedeutung besitzen, das man isoliert und gewünschtenfalls in ein Salz überführt.

2. Verfahren gemäß Anspruch 1, dadurch gekennzeichnet, daß man von einem Produkt der Formel (II) ausgeht, worin X ein Chlor- oder Bromatom bedeutet, und daß man in Gegenwart eines Wasserstoffsäure bindenden Mittels arbeitet.

3. Verfahren gemäß Anspruch 1, dadurch gekennzeichnet, daß man von einem Produkt der Formel (II) ausgeht, worin X einen Alkoxyrest mit 1 bis 5 Kohlenstoffatomen bedeutet, und daß man in Gegenwart eines Alkylaluminium-Derivats arbeitet.

4. Verfahren gemäß einem der Ansprüche 1 bis 3, dadurch gekennzeichnet, daß man von einem Produkt der Formel (II) ausgeht, worin R ein Wasserstoffatom oder einen Methylrest bedeutet, $R_1$ ein Wasserstoff- oder Chloratom oder einen Methoxyrest darstellt oder $R_1$ in 6-Stellung ein Fluoratom oder einen Methyl-, Ethyl-, Isopropyl-, Methylthio- oder Trifluormethyl-Rest bedeutet.

5. Verfahren gemäß Anspruch 4, dadurch gekennzeichnet, daß man von einem Produkt der Formel (II) ausgeht, worin R ein Wasserstoffatom bedeutet.

6. Verfahren gemäß einem der Ansprüche 1 bis 5, dadurch gekennzeichnet, daß man von einem Produkt der Formel (II) ausgeht, worin R und $R_1$ ein Wasserstoffatom bedeuten.

7. Verfahren gemäß einem der Ansprüche 1 bis 5, dadurch gekennzeichnet, daß man von einem Produkt der Formel (II) ausgeht, worin R ein Wasserstoffatom bedeutet und $R_1$ einen Ethylrest in 6-Stellung darstellt.

8. Verfahren gemäß einem der Ansprüche 1 bis 5, dadurch gekennzeichnet, daß man von einem Produkt der Formel (II) ausgeht, worin R ein Wasserstoffatom bedeutet und $R_1$ ein Chloratom in 6-Stellung darstellt.

9. Verfahren gemäß einem der Ansprüche 1 bis 5, dadurch gekennzeichnet, daß man von einem Produkt der Formel (II) ausgeht, worin R einen Methylrest bedeutet und $R_1$ ein Wasserstoffatom darstellt.